(19) Europäisches Patentamt

European Patent Office

Office européen des brevets

(11) EP 1 696 044 A1

(12) EUROPÄISCHE PATENTANMELDUNG

(43) Veröffentlichungstag:
30.08.2006 Patentblatt 2006/35

(51) Int Cl.:
*C22C 19/07* (2006.01)     *A61K 6/04* (2006.01)

(21) Anmeldenummer: 05101162.5

(22) Anmeldetag: 16.02.2005

(84) Benannte Vertragsstaaten:
AT BE BG CH CY CZ DE DK EE ES FI FR GB GR
HU IE IS IT LI LT LU MC NL PL PT RO SE SI SK TR
Benannte Erstreckungsstaaten:
AL BA HR LV MK YU

(71) Anmelder: BEGO Bremer Goldschlägerei Wilh.
Herbst GmbH & Co.
KG
28359 Bremen (DE)

(72) Erfinder: Strietzel, Roland
28865, Lilienthal (DE)

(74) Vertreter: Eisenführ, Speiser & Partner
Patentanwälte Rechtsanwälte
Postfach 10 60 78
28060 Bremen (DE)

(54) **Aufbrennfähige Legierung zur Herstellung keramisch verblendeter Dentalrestaurationen**

(57)     Beschrieben wird eine aufbrennfähige Legierung zur Herstellung keramisch verblendeter Dentalrestaurationen, umfassend oder bestehend aus:

| | |
|---|---|
| Cobalt | 55-65 Gewichtsprozent, |
| Chrom | 20-30 Gewichtsprozent, |
| Wolfram und/oder Molybdän | wobei die aus dem Gewichtsanteil an Molybdän und dem halben Gewichtsanteil an Wolfram gebildete Summe im Bereich von 4-12 Gewichtsprozent liegt, |
| Gallium | 2-4 Gewichtsprozent, |
| Silizium | 0-2 Gewichtsprozent, |
| Mangan | 0,05-1,9 Gewichtsprozent, |
| Stickstoff | 0-0,4 Gewichtsprozent, |
| Kohlenstoff | 0-0,02 Gewichtsprozent, |
| Vanadium, Niob, Tantal, Eisen, Titan, Zirkonium, Hafnium | insgesamt 0-5 Gewichtsprozent, |
| Nickel | 0-0,1 Gewichtsprozent, |
| Platingruppenmetalle, Rhenium, Gold, Silber, Kupfer | insgesamt 0-0,09 Gewichtsprozent, |
| sonstige Metalle, Halbmetalle und Verunreinigungen | 0-1 Gewichtsprozent, |

wobei die Gewichtsprozentangaben jeweils bezogen sind auf das Gesamtgewicht der Legierung.

EP 1 696 044 A1

**Beschreibung**

Die Erfindung betrifft eine aufbrennfähige Legierung zur Herstellung keramisch verblendeter Dentalrestaurationen, beispielsweise für die Herstellung von Kronen, Brücken, Einlagen und anderen Zahnprothesen, die mit einer Keramikoberfläche versehen werden sollen.

Bei der erfindungsgemäßen Legierung handelt es sich um eine edelmetallfreie, korrosionsbeständige Legierung auf Cobalt-Chrom-Basis (CoCr-Basis).

Legierungen auf Cobalt-Chrom-Basis sind beispielsweise aus den folgenden Veröffentlichungen bekannt:

DE 102 31 737 C1, DE 198 45 638 C1, WO 02/36080, WO 00/64403, EP 1 173 136 A1, DE 102 26 221 C1, DE 22 25 577 C3, DE 31 09 053, DE 34 36 118 C1, DE 36 24 377, DE 39 41 820 C2, EP 0 804 934 B1, US 3 366 478, DE 37 44 491 C1, DE 198 15 091 A1, FR 2 750 858 A1, FR 2 750 867 A1, DE 30 38 036 A1, DE 41 23 606 A1, DE 102 52 776 A1, WO 2004/042098 A1, DE 30 01 126 A1, DE 35 10 331 C1 und US 4 263 045:

Auch im Handel sind eine Reihe von aufbrennfähigen Legierungen auf Cobalt-Chrom-Basis erhältlich.

Aufbrennfähige Cobalt-Chrom-Legierungen werden in der Praxis häufig mit Keramiken verblendet, deren linearer Wärmeausdehnungskoeffizient (WAK-Wert) im Bereich von etwa 13-15 [$10^{-6}$ $K^{-1}$] im Bereich von 25 -500 °C liegt. Auch die erfindungsgemäße aufbrennfähige Legierung ist zur Verblendung mit derartigen Verblendkeramiken vorgesehen.

Bei der Konzeption neuer aufbrennfähiger Legierungen auf Cobalt-Chrom-Basis muss der Fachmann eine Vielzahl technischer Eigenschaften berücksichtigen und versuchen, ausgewählte Eigenschaften besonders günstig einzustellen, ohne dabei die anderen Eigenschaften in besonders nachteiliger Weise zu beeinflussen.

Im Zusammenhang mit der Verwendung üblicher Legierungen auf Cobalt-Chrom-Basis wurde es bislang von den Fachleuten häufig als nachteilig empfunden, dass der richtige Gießzeitpunkt nicht mit ausreichender Sicherheit erkannt werden kann; deshalb werden bereits in größerem Umfang automatische Gießzeitpunkt-Erkennungssysteme als Hilfsmittel eingesetzt.

Zudem hat es sich bei der Verwendung handelsüblicher CoCr-Legierungen häufig als nachteilig erwiesen, dass aus der Legierung hergestellte Brücken mit großer Spannweite, z.B. 14-gliedrige Brücken, häufig eine nur unzureichende Passung aufweisen. Es ist deshalb häufig eine aufwendige Nachbearbeitung erforderlich, die insbesondere mit Trenn- und Fügeoperationen verbunden ist.

Ferner wird seitens der Hersteller von aufbrennfähigen CoCr-Legierungen bzw. damit kompatiblen Verblendkeramiken regelmäßig eine Verfahrensgestaltung empfohlen, bei der das keramisch verblendete Gerüst (Dentalrestauration) nach dem Sintern einer Langzeitabkühlung unterzogen wird, um den WAK der Keramik in geeigneter Weise zu steuern. Der WAK einer Dentalkeramik erhöht sich nämlich (auf Grund des Wachstums von Leuzitkristallen), wenn die Abkühlphase zeitlich gestreckt wird. In der Praxis ist es günstig, wenn der WAK der Keramik etwas niedriger ist als der WAK der zu verblendenden aufbrennfähigen Legierung. Auf diese Weise kann sich nach dem Abkühlen in der Dentalkeramik eine erforderliche Druckspannung aufbauen. Sind die Wärmeausdehnungskoeffizienten der Legierung und der Dentalkeramik nicht optimal aufeinander abgestimmt, kommt es häufig zu Sprüngen oder Abplatzungen in der Keramik. Wird jedoch eine Langzeitabkühlung durchgeführt, um die WAK-Werte von Legierung und Keramik aufeinander abzustimmen, so ist dies naturgemäß nachteilig, weil der Zahntechniker nun eine längere Zeit bis zum nächsten Bearbeitungsschritt warten muss. Pro Brennzyklus dauert eine Langzeitabkühlung etwa 10 Minuten länger als eine "normale" Abkühlung.

Angesichts der vorstehend beschriebenen Probleme bei der Verwendung herkömmlicher CoCr-Legierungen war es die der vorliegenden Erfindung zu Grunde liegende Aufgabe, eine CoCr-Legierung anzugeben, (i) bei der der Gießzeitpunkt eindeutig erkennbar ist, (ii) die nach dem Abguss eine besondere gute Passung aufweist und (iii) bei der es nach dem Aufbrennen einer Verblendkeramik nicht erforderlich ist, eine Langzeitabkühlung durchzuführen.

Dabei sollten die sonstigen für eine aufbrennfähige Dentallegierung wichtigen Legierungsmerkmale innerhalb der vom Fachmann bevorzugten Bereiche liegen.

Erfindungsgemäß wird diese Aufgabe gelöst durch eine aufbrennfähige Legierung zur Herstellung keramisch verblendeter Dentalrestaurationen, umfassend oder bestehend aus:

| | |
|---|---|
| Cobalt | 55-65 Gewichtsprozent, |
| Chrom | 20-30 Gewichtsprozent |
| Wolfram und/oder Molybdän | wobei die aus dem Gewichtsanteil an Molybdän und dem halben Gewichtsanteil an Wolfram gebildete Summe im Bereich von 4-12 Gewichtsprozent liegt, |
| Gallium | 2-4 Gewichtsprozent, |

(fortgesetzt)

| | |
|---|---|
| Silizium | 0-2 Gewichtsprozent, |
| Mangan | 0,05-1,9 Gewichtsprozent, |
| Stickstoff | 0-0,4 Gewichtsprozent, |
| Kohlenstoff | 0-0,02 Gewichtsprozent, |
| Vanadium, Niob, Tantal, Eisen, Titan, Zirkonium, Hafnium | insgesamt 0-5 Gewichtsprozent, |
| Nickel | 0-0,1 Gewichtsprozent, |
| Platingruppenmetalle, Rhenium, Gold, Silber, Kupfer | insgesamt 0-0,09 Gewichtsprozent, |
| sonstige Metalle, Halbmetalle und Verunreinigungen | 0-1 Gewichtsprozent, |

wobei die Gewichtsprozentangaben (hier wie nachfolgend) jeweils bezogen sind auf das Gesamtgewicht der Legierung.

Die erfindungsgemäße aufbrennfähige Legierung umfasst einen Anteil von 55-65 Gewichtsprozent Cobalt. Es hat sich gezeigt, dass ein höherer Anteil an Cobalt zu einer unerwünscht reduzierten Festigkeit führen würde; ebenso würde ein kleinerer Anteil an Cobalt zumindest bei entsprechender Erhöhung des Chrom-Anteiles zu einer Legierung führen, die in unerwünschter Weise versprödet.

Vorzugsweise beträgt der Anteil an Cobalt in der erfindungsgemäßen Legierung 58-62 Gewichtsprozent, bevorzugt 59,7-60,7 Gewichtsprozent.

In der erfindungsgemäßen Legierung beträgt der Anteil an Chrom 20-30 Gewichtsprozent. Es hat sich in eigenen Untersuchungen gezeigt, dass ein Cr-Anteil von weniger als 20 Gewichtsprozent häufig zu einer inakzeptablen hohen Korrosivität der entsprechenden Legierung führt und daher zu deren Löslichkeit in der Mundhöhle. Ein Anteil von mehr als 30 Gewichtsprozent führt hingegen zu einer Legierung, die in inakzeptabler Weise versprödet.

Vorzugsweise liegt der Anteil an Chrom in der erfindungsgemäßen Legierung im Bereich von 23-27 Gewichtsprozent, besonders bevorzugt ist der Bereich von 24,5-25,5 Gewichtsprozent.

Die erfindungsgemäße Legierung umfasst Wolfram und/oder Molybdän. Dabei liegt die aus dem Gewichtsanteil an Molybdän und dem halben Gewichtsanteil an Wolfram gebildete Summe im Bereich von 4-12 Gewichtsprozent. In diesem Bereich wird ein guter Kompromiss zwischen Korrosionsfestigkeit und mechanischen Eigenschaften wie Härte, Festigkeit und Sprödigkeit gefunden.

Unter Berücksichtigung des Anteils an Chrom in einer erfindungsgemäßen Legierung ergibt sich die folgende Beziehung:

$$[Cr] + 3,3 \, (0,5 \, [W] + [Mo]) > 33,2.$$

Dieser Wert liegt beträchtlich über dem gemäß DIN 13912 geforderten Wert für die Wirksumme von 30.

Vorzugsweise liegt in einer erfindungsgemäßen Legierung der Anteil an Wolfram im Bereich von 0-14 Gewichtsprozent und/oder der Anteil an Molybdän im Bereich von 0-10 Gewichtsprozent. Zur Einstellung einer vorteilhaften niedrigen Härte wird das Gewichtsverhältnis W : Mo vorzugsweise so eingestellt, dass es größer ist als 1:2.

Vorzugsweise ist der Anteil an Molybdän in einer erfindungsgemäßen Legierung größer als 3,8 Gewichtsprozent, denn so lässt sich eine hohe Korrosionsresistenz erreichen.

Der Anteil an Wolfram in einer erfindungsgemäßen Legierung ist vorteilhafterweise größer als 3 Gewichtsprozent, und zwar insbesondere dann, wenn der Anteil an Molybdän größer als 3,8 Gewichtsprozent ist. Das Wolfram bewirkt in diesem Fall eine gewünscht niedrige Härte.

Bevorzugte erfindungsgemäße Legierungen besitzen eine (recht niedrige) Vickershärte (HV10) im Bereich von 260-300, eine Härte HV10 von 275-285 ist dabei bevorzugt.

Eine gewünscht niedrige Härte und eine besonders hohe Korrosionsresistenz lässt sich insbesondere mit einer erfindungsgemäßen Legierung erreichen, wenn diese 4,8-8,2 Gewichtsprozent Wolfram und 3,8-5,8 Molybdän umfasst.

Der Anteil an Gallium in einer erfindungsgemäßen Legierung liegt im Bereich von 2-4 Gewichtsprozent. In diesem Mengenbereich trägt Gallium zu einer niedrigen Härte bei, ohne die Korrosionsfestigkeit nennenswert zu reduzieren. Überdies lässt sich mit einer erfindungsgemäßen Legierung auf Grund des erfindungsgemäß vorhandenen Anteils an Gallium ein günstiger Haftverbund zu üblichen Verblendkeramiken herstellen.

Ein Anteil an Gallium von mehr als 4 Gewichtsprozent würde hingegen zu einer inakzeptabel hohen Sprödigkeit des fertigen Produktes (Gussstückes) und beim zahntechnischen Guss zur Bildung von Oxidschichten auf der Schmelze führen, welche die Bestimmung des Gießzeitpunktes erschweren würde. Bei sehr viel höheren als den erfindungsgemäß vorhandenen Anteilen an Gallium würde sich zudem mehrphasige Gefüge ausbilden. Auch würde bei einem zu hohen Anteil an Gallium der Wärmeausdehnungskoeffizient einer entsprechenden Legierung in vielen Fällen zu hoch sein, was eine Inkompatibilität mit üblichen Dentalkeramiken nach sich ziehen würde.

Der Einsatz von weniger als 2 Gewichtsprozent Gallium würde nicht mehr zu den oben genannten, gewünschten Effekten (Beeinflussung von Härte, Korrosionsfestigkeit und Haftverbund) führen.

Vorzugsweise liegt der Anteil an Gallium in einer erfindungsgemäßen Legierung im Bereich von 2,5-3,3 Gewichtsprozent.

Der Anteil an Silizium einer erfindungsgemäßen Legierung liegt im Bereich von 0-2 Gewichtsprozent. Eine Erhöhung des Anteils auf einen Wert größer 2 Gewichtsprozent würde zu einer drastischen Erhöhung der Sprödigkeit führen und eventuell zur Ausbildung unerwünschter Eutektika. Innerhalb des Bereiches von 0-2 Gewichtsprozent wirkt das Silizium jedoch in gewünschter Weise als Sauerstofffänger. Zudem trägt es dazu bei, dass das Schmelzintervall der erfindungsgemäßen Legierung auf einer vergleichsweisen niedrigen Temperatur liegt und die Viskosität einer Schmelze der erfindungsgemäßen Legierung vergleichsweise niedrig ist.

Vorzugsweise liegt der Anteil an Silizium in einer erfindungsgemäßen Legierung im Bereich von 0,3-2 Gewichtsprozent. Der Einsatz von weniger als 0,3 Gewichtsprozent Silizium würde dazu führen, dass die vorstehend genannten Funktionen des Siliziums in einer erfindungsgemäßen Legierung im Einzelfall nicht mehr zur vollen Zufriedenheit erreicht werden können.

Der Anteil an Mangan in einer erfindungsgemäßen Legierung liegt im Bereich vom 0,05-1,9 Gewichtsprozent. Mangan fungiert in einer erfindungsgemäßen Legierung als Sauerstofffänger, Haftoxidbildner und Entschwefelungsmittel. Zudem trägt Mangan dazu bei, dass die Viskosität einer Schmelze einer erfindungsgemäßen Legierung recht niedrig ist.

Ein Anteil an Mangan von mehr als 1,9 Gewichtsprozent würde zu einer unerwünschten Erhöhung des WAK-Wertes der entsprechenden Legierung und zu einem ungünstigeren Gießverhalten führen; insbesondere zeigen Gussstücke, die aus einer Vergleichslegierung abgegossen wurden, welche einen Anteil an Mangan von mehr als 1,9 Gewichtsprozent besaß, eine unerwünscht raue Oberfläche.

Ein Anteil an Mangan von weniger als 0,05 Gewichtsprozent würde dazu führen, dass die vorstehend genannten Funktionen des Mangans in einer entsprechenden Legierung im Einzelfall nicht mehr zur vollen Zufriedenheit erreicht würden.

Vorzugsweise beträgt der Anteil an Mangan in einer erfindungsgemäßen Legierung 0,05-1 Gewichtsprozent.

Die erfindungsgemäße Legierung umfasst in jedem Falle Cobalt, Chrom und Gallium sowie zumindest ein Metall aus der Gruppe bestehend aus Wolfram und Molybdän. Andere Bestandteile sind optional.

Der Anteil an Stickstoff in einer erfindungsgemäßen Legierung liegt im Bereich von 0-0,4 Gewichtsprozent. In einer erfindungsgemäßen Legierung ist somit ein geringer Anteil an Stickstoff tolerabel. Vorhandene Stickstoffmengen in einer erfindungsgemäßen Legierung führen zu einer recht hohen mechanischen Festigkeit.

Der Anteil an Kohlenstoff in einer erfindungsgemäßen Legierung liegt im Bereich von 0-0,02 Gewichtsprozent. Derartig kleine Konzentrationen an Kohlenstoff sind in einer erfindungsgemäßen Legierung tolerabel; höhere Anteile würden jedoch häufig stören; z.B. führen höhere Kohlenstoff-Anteile beim Laserschweißen (z.B. während eines Reparaturvorganges) häufig zu einer Versprödung der entstehenden Schweißnaht.

In einer erfindungsgemäßen Legierung liegt der Gesamtanteil an Tantal, Eisen, Titan, Zirkonium und Hafnium im Bereich von 0-5 Gewichtsprozent. Kleinere Mengen der genannten Elemente sind somit in einer erfindungsgemäßen Legierung tolerabel, eine Gesamtmenge an den genannten Verbindungen von mehr als 5 Gewichtsprozent würde jedoch eine unerwünschte Aufhärtung (Erhöhung der Härte) und Erhöhung der Sprödigkeit nach sich ziehen.

Vorzugsweise beträgt der Anteil jedes Einzelnen der Elemente Vanadium, Niob, Tantal, Eisen, Titan, Zirkonium und Hafnium in einer erfindungsgemäßen Legierung weniger als 1 Gewichtsprozent.

Vorzugsweise ist der Anteil an Tantal in einer erfindungsgemäßen Legierung kleiner als 0,18 Gewichtsprozent.

Der Anteil an Nickel in einer erfindungsgemäßen Legierung liegt im Bereich von 0-0,1 Gewichtsprozent. Höhere Anteile an Nickel würden die Gefahr von Kontaktallergien erhöhen.

Der Anteil an Platingruppenmetallen, Rhenium, Gold, Silber und Kupfer in einer erfindungsgemäßen Legierung liegt im Bereich von 0-0,9 Gewichtsprozent.

Sonstige Metalle, Halbmetalle und Verunreinigungen können in einer erfindungsgemäßen Legierung im Bereich von 0-1 Gewichtsprozent vorhanden sein.

In bevorzugten erfindungsgemäßen Legierungen fällt der Gewichtsanteil von Legierungsbestandteilen (soweit vorhanden) in der folgenden Reihenfolge ab: Cobalt, Chrom, Wolfram, Molybdän, Gallium, Silizium, Mangan.

Eine bevorzugte erfindungsgemäße Legierung umfasst nicht:

Stickstoff und/oder Kohlenstoff und/oder Vanadium und/oder Niob und/oder Tantal und/oder Eisen und/oder Titan und/oder Zirkonium und/oder Hafnium und/oder Nickel und/oder Metalle der Platingruppe und/oder Rhenium und/oder Gold und/oder Silber und/oder Kupfer.

Besonders vorteilhaft ist es, wenn keines der zuletzt genannten Elemente in der erfindungsgemäßen Legierung vorhanden ist.

Besonders bevorzugte erfindungsgemäße Legierungen bestehen aus:

| | |
|---|---|
| Cobalt | 60,2 ± 2 Gewichtsprozent, |
| Chrom | 25,0 ± 2 Gewichtsprozent, |
| Wolfram | 6,2 ± 1 Gewichtsprozent, |
| Molybdän | 4,8 ± Gewichtsprozent, |
| Gallium | 2,9 ± 1 Gewichtsprozent, |
| Silizium | 0,8 ± 0,3 Gewichtsprozent, |
| Mangan | 0,1 ± 0,03 Gewichtsprozent und |
| sonstige Bestandteile | 0-1 Gewichtsprozent. |

Ein ganz bevorzugtes Ausführungsbeispiel für eine erfindungsgemäße Legierung besitzt die folgende Zusammensetzung:

| | |
|---|---|
| Cobalt | 60,2 Gewichtsprozent, |
| Chrom | 25,0 Gewichtsprozent, |
| Wolfram | 6,2 Gewichtsprozent, |
| Molybdän | 4,8 Gewichtsprozent, |
| Gallium | 2,9 Gewichtsprozent, |
| Silizium | 0,8 Gewichtsprozent, |
| Mangan | 0,1 Gewichtsprozent. |

Für die besonders bevorzugte erfindungsgemäße Legierung wurden folgende Legierungseigenschaften bestimmt:

| | |
|---|---|
| Dichte [g/cm$^3$] | 8,5 |
| Vickershärte [HV 10] | 280 |
| Elastizitätsmodul [GPa] | ca. 220 |
| Dehngrenze ($R_p$ 0,2) [MPa] | 540 |
| Zugfestigkeit [MPa] | 680 |
| Bruchdehnung [A5] [%] | 14 |
| Schmelzintervall [°C] | 1360-1400 |
| Gießtemperatur [°C] | ca. 1500 |
| WAK [$10^{-6}K^{-1}$] 25-500°C | 14,0 |
| WAK [$10^{-6}K^{-1}$] 25-600°C | 14,2. |

Die erfindungsgemäße Legierung, insbesondere in einer bevorzugten Ausgestaltung, zeichnet sich durch die folgenden Eigenschaften besonders positiv aus:

- keine Langzeitabkühlung beim keramischen Verblenden erforderlich, d.h. die Verarbeitungszeit ist niedriger als bei der Mehrzahl üblicher CoCr-Legierungen;

- hohe Passgenauigkeit der aus der Legierung gegossenen Gerüste (Dentalrestaurationen);

- vergleichsweise niedrige Härte, d.h. besonders gute Be/Verarbeitungseigenschaften;

- günstige Laserschweißbarkeit;

- hohe Korrosionsresistenz;

- günstige Gießzeitpunkterkennung (Zahntechniker sieht, wann er den Guss auslösen muss);

- Legierung bildet dünnflüssige Schmelze;

- Schmelze der erfindungsgemäßen Legierung besitzt günstiges Ausfließverhalten (daraus resultiert ein hohes Formfüllungsvermögen der Schmelze);

- Legierung ist verblendbar mit allen gängigen Verblendkunststoffen;

- Legierung ist verblendbar mit Verblendkeramiken, die einen WAK im Bereich 13-15 [$10^{-6}K^{-1}$] besitzen.

Beim Verblenden mit Keramik besitzt die erfindungsgemäße Legierung die folgenden Vorteile:

- günstiger WAK;

- günstige chemische Haftung (vermutlich deshalb, weil neben Chrom auch Gallium als Haftoxidbildner wirkt);

- hohe Warmfestigkeit (ein Gerüst aus der erfindungsgemäßen Legierung verzieht sich nicht während der Brände);

- alle marktüblichen Keramiken mit einem WAK im Bereich 13-15 [$10^{-6}$ $K^{-1}$] sind verwendbar;

- "normale" Abkühlung (keine Langzeitabkühlung) für Brücken aller Spannen (bis hin zu 14-gliedrigen Brücken).

Die Erfindung betrifft auch eine keramisch verblendete Dentalrestauration, umfassend:

- ein Dentalgerüst aus einer erfindungsgemäßen Legierung sowie

- eine auf das Dentalgerüst aufgebrannte Dentalkeramik mit einem WAK im Bereich von 13-15 [$10^{-6}K^{-1}$].

Nachfolgend wird die Erfindung anhand eines Ausführungsbeispiels näher beschreiben:
Ausführungsbeispiel:
1. Herstellung eines 14-gliedrigen Brückengerüstes:
Es wurde aus Wachs eine 14-gliedrige Oberkiefer-Brücke modelliert; als Modell diente dabei eine reale Patientensituation. Die Mindest-Wandstärke war jeweils 0,3 mm. Es wurden die anatomischen Formen berücksichtigt, so dass der Hauptanteil der Restauration später, nach dem Guss, aus Metall bestand.
Die hergestellte Wachsmodellation wurde in eine phosphatgebundene Einbettmasse eingebettet.
Die resultierende Muffel wurde dann auf eine Temperatur von 900 °C gebracht (Vorwärmtemperatur) und dort für 60 min gehalten.
Der Guss erfolgte in einer induktionsgeheizten Vakuum-Druck-Gussmaschine (Nautilus® T/Fa. BEGO, Programm 6004) unter Verwendung von Ingots einer erfindungsgemäßen Legierung der folgenden Zusammensetzung:

| Cobalt | 60,2 Gewichtsprozent, |
|---|---|
| Chrom | 25,0 Gewichtsprozent, |
| Wolfram | 6,2 Gewichtsprozent, |
| Molybdän | 4,8 Gewichtsprozent, |
| Gallium | 2,9 Gewichtsprozent, |
| Silizium | 0,8 Gewichtsprozent, |
| Mangan | 0,1 Gewichtsprozent. |

Die Ingots wurden in üblicher Weise erhitzt. Der Guß wurde ca. 4 s nach dem Eintauchen der letzten festen Bestandteile der Ingots in die Schmelze manuell ausgelöst. Der Gießzeitpunkt war sehr günstig zu erkennen, da eine zwischenzeitlich vorhandene Oxidhaut schnell und eindeutig aufriss.
Nach dem Abkühlen der Muffel wurde die Einbettmasse grob mechanisch entfernt. Danach wurde das erhaltene Brückengerüst mit Korund der Körnung 250 $\mu$m (Korox® 250/Fa. BEGO) bei 3 bar abgestrahlt. Abschließend wurde die Oberfläche des Brückengerüstes mit einer feinverzahnten Hartmetallfräse bearbeitet. Aufgrund der niedrigen Härte und der guten Spanbarkeit der eingesetzten Legierung gestaltete sich das Ausarbeiten sehr angenehm für den Zahntechniker. Die Passung des Gerüstes war im Vergleich mit den Erfahrungen aus dem Abguss anderer CoCr-Legierungen auffällig gut.

Anmerkung:      Bei CoCr-Legierungen ist man gewöhnlicherweise ein mehr oder weniger starkes Schaukeln, welches durch Passungsfehler bedingt ist, gewohnt. Dieses war bei insgesamt drei hergestellten Brücken zwei mal sehr gering und einmal nicht zu beobachten. Die Passung der beiden schaukelnden Brücken wurde durch Trennen und Fügen wieder beseitigt. Dazu wurde einmal das Laserschweißen (Zulegematerial: Wiroweld/Fa. BEGO) und einmal das Löten (mit Wirobond®-Lot/Fa. BEGO) verwendet. Beide Fügetechniken lassen sich problemlos anwenden und sind genauso wie bei den bisher erhältlichen CoCr-Legierungen durchzuführen. Die Festigkeit der Fügestellen wurde in zusätzlichen Versuchen gemäß DIN 13972-2:2002 (Laserschweißbarkeit), bzw. ISO 9333:1990 (Lötung) untersucht. Die Anforderungen der Normen wurden jedes Mal erfüllt bzw. deutlich übertroffen.

2. Verblenden des Brückengerüstes mit Dentalkeramik mittels Wash- und Grundmassebrand:

Vor dem keramischen Verblenden wurde die Oberfläche des Brückengerüstes (aus 1. oben) nochmals, so wie unter 1. beschrieben, abgestrahlt und abgedampft, um die Oberfläche für einen folgenden Washbrand zu konditionieren.

Der Washbrand erfolgte nach Auftrag einer dünnen Suspension einer Verblendkeramik des Typs Omega 900 (Fa. Vita). Der Auftrag war dabei nicht deckend.

Es wurde dann nach Auftrag einer deckenden Schicht Pulver-Opaker des Typs Omega 900 (Fa. Vita) ein Grundmasse-Brand durchgeführt.

Für die Durchführung des Wash- und des Grundmasse-Brandes wurde, soweit hier nicht anders angegeben, gemäß der Verarbeitungsanleitung des Keramik-Herstellers (Vita) verfahren. Es wurden die Temperaturen und Zeiten verwendet, die in der unten stehenden Tabelle angegeben sind. Als Brennofen diente ein Vakumat 300 (Fa. Vita).

Auf eine langsame Abkühlung wurde verzichtet und eine normale (d.h. vergleichsweise schnelle) Abkühlung durchgeführt. Trotz der normalen Abkühlung traten überraschenderweise keine Sprünge oder Abplatzungen auf, auch nicht nach längerem Liegenlassen (über 3 Tage). Durch die normale Abkühlung konnte der Zahntechniker pro Brand ca. 10 min Zeit einsparen. Der Einsatz der unter 1. angegebenen Legierung ermöglicht somit ein sehr ökonomisches Arbeiten. Im beschriebenen Verfahren wurde auf einen Oxidbrand (vor dem Washbrand) verzichtet. Ein solcher kann aber ergänzend durchgeführt werden, um die Qualität der Oberfläche zu überprüfen. Im Falle einer ausreichenden Oberflächenqualität dürfen dann keine Schattierungen zu erkennen sein, die Oxidschicht muss vielmehr eine einheitliche Farbe aufweisen. Vor den folgenden Bränden muß die Oxidschicht wieder sorgfältig durch Abstrahlen entfernt werden.

Auf Brände des Typs "Schultermassebrand mit Margin" und "Glanzbrand mit Akzentfluid" (nach dem Grundmassebrand) wurde im Rahmen des Ausführungsbeispieles verzichtet. Solche Brände können aber ergänzend durchgeführt werden. Gemäß der nachfolgenden Tabelle wurden ergänzend folgende Brände durchgeführt: 1. Dentinbrand, 2. Dentinbrand, Korrekturbrand und Glanzbrand. Dabei wurden wieder Keramikmaterialien des Typs Omega 900 (Fa. Vita) eingesetzt. Die Verbundfestigkeit wurde in In-vitro Versuchen (Abschlag-Test, Abschreck-Test und Biegeversuch gemäß DIN EN ISO 9693:2000) bestimmt. Dabei wurden sämtliche Anforderungen deutlich übertroffen.

Die unverblendeten Anteile (Kronenränder, aber auch unverblendete Kronen) ließen sich sehr einfach polieren. Der schnell erreichte Glanz erfüllt alle Ansprüche an die Ästhetik und bietet dem Anheften von z. B. Speiseresten und der Plaquebildung großen Widerstand entgegen.

Tabelle:

| Brand | Vorwärm-temperatur [°C] | Haltezeit [min] | Heizzeit [min] | Aufheizrate [°C/min] | Endtemperatur [°C] | Haltezeit [min] | Gesamt-vakuum-zeit [min] |
|---|---|---|---|---|---|---|---|
| Washbrand | 600 | 2 | 4 | 75 | 900 | 2 | 4 |
| Grundmasse | 600 | 2 | 4 | 75 | 900 | 1 | 4 |
| 1. Dentinbrand | 600 | 6 | 6 | 50 | 900 | 1 | 6 |
| 2. Dentinbrand | 600 | 6 | 6 | 50 | 890 | 1 | 6 |
| Korrekturbrand | 600 | 4 | 6 | 33 | 800 | 1 | 6 |
| Glanzbrand | 600 | - | 4 | 75 | 900 | 2 | - |

**Patentansprüche**

1. Aufbrennfähige Legierung zur Herstellung keramisch verblendeter Dentalrestaurationen, umfassend oder bestehend aus:

Cobalt                                                                55-65 Gewichtsprozent,

(fortgesetzt)

| | |
|---|---|
| Chrom | 20-30 Gewichtsprozent, |
| Wolfram und/oder Molybdän | wobei die aus dem Gewichtsanteil an Molybdän und dem halben Gewichtsanteil an Wolfram gebildete Summe im Bereich von 4-12 Gewichtsprozent liegt, |
| Gallium | 2-4 Gewichtsprozent, |
| Silizium | 0-2 Gewichtsprozent, |
| Mangan | 0,05-1,9 Gewichtsprozent, |
| Stickstoff | 0-0,4 Gewichtsprozent, |
| Kohlenstoff | 0-0,02 Gewichtsprozent, |
| Vanadium, Niob, Tantal, Eisen, Titan, Zirkonium, Hafnium | insgesamt 0-5 Gewichtsprozent, |
| Nickel | 0-0,1 Gewichtsprozent, |
| Platingruppenmetalle, Rhenium, Gold, Silber, Kupfer | insgesamt 0-0,09 Gewichtsprozent, |
| sonstige Metalle, Halbmetalle und Verunreinigungen | 0-1 Gewichtsprozent, |

wobei die Gewichtsprozentangaben jeweils bezogen sind auf das Gesamtgewicht der Legierung.

2. Legierung nach Anspruch 1, umfassend:

| | |
|---|---|
| Cobalt und/oder | 58-62 Gewichtsprozent |
| Chrom und/oder | 23-27 Gewichtsprozent |
| Wolfram | 0-14 Gewichtsprozent |
| und/oder | |
| Molybdän | 0-10 Gewichtsprozent |
| und/oder | |
| Gallium | 2,5-3,3 Gewichtsprozent |
| und/oder | |
| Silizium | 0,3-2 Gewichtsprozent |
| und/oder | |
| Mangan und/oder | 0,05-1 Gewichtsprozent |
| Vanadium, Niob, Tantal, Eisen, Titan, Zirkonium, Hafnium | jeweils weniger als 1 Gewichtsprozent. |

3. Legierung nach einem der Ansprüche 1 oder 2, umfassend:

| | |
|---|---|
| Cobalt und/oder | 59,7-60,7 Gewichtsprozent |
| Chrom und/oder | 24,5-25,5 Gewichtsprozent |

Wolfram und Molybdän, wobei das Gewichtsverhältnis W: Mo größer ist als 1:2.

4. Legierung nach einem der Ansprüche 1-3, umfassend:

| | |
|---|---|
| Molybdän und/oder | mehr als 3,8 Gewichtsprozent |
| Wolfram | mehr als 3 Gewichtsprozent |

5. Legierung nach einem der Ansprüche 1-4, umfassend

| | |
|---|---|
| Wolfram und | 4,2-8,2 Gewichtsprozent |
| Molybdän | 3,8-5,8 Gewichtsprozent. |

**6.** Legierung nach einem der Ansprüche 1-5, wobei der Gewichtsanteil von Legierungsbestandteilen, soweit diese vorhanden sind, in folgender Reihenfolge abfällt:

Cobalt, Chrom, Wolfram, Molybdän, Gallium, Silizium, Mangan.

**7.** Legierung nach einem der Ansprüche 1-6, nicht umfassend:

Stickstoff und/oder Kohlenstoff und/oder Vanadium und/oder Niob und/oder Tantal und/oder Eisen und/oder Titan und/oder Zirkonium und/oder Hafnium und/oder Nickel und/oder Metalle der Platingruppe und/oder Rhenium und/oder Gold und/oder Silber und/oder Kupfer.

**8.** Legierung nach einem der vorrangehenden Ansprüche, bestehend aus:

| | |
|---|---|
| Cobalt | 60,2 $\pm$ 2 Gewichtsprozent, |
| Chrom | 25,0 $\pm$ 2 Gewichtsprozent, |
| Wolfram | 6,2 $\pm$ 1 Gewichtsprozent, |
| Molybdän | 4,8 $\pm$ Gewichtsprozent, |
| Gallium | 2,9 $\pm$ 1 Gewichtsprozent, |
| Silizium | 0,8 $\pm$ 0,3 Gewichtsprozent, |
| Mangan | 0,1 $\pm$ 0,03 Gewichtsprozent und |
| sonstige Bestandteile | 0-1 Gewichtsprozent. |

**9.** Keramisch verblendete Dentalrestauration, umfassend:

- ein Dentalgerüst aus einer Legierung nach einem der Ansprüche 1-6 sowie
- eine auf das Dentalgerüst aufgebrannte Dentalkeramik mit einem WAK im Bereich von 13-15 [$10^{-6}K^{-1}$].

**Europäisches Patentamt**

**EUROPÄISCHER RECHERCHENBERICHT**

Nummer der Anmeldung

EP 05 10 1162

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | DE 299 09 031 U1 (AHLDEN, MANFRED) 5. August 1999 (1999-08-05) * das ganze Dokument * ----- | 1-9 | C22C19/07 A61K6/04 |
| A,D | WO 2004/042098 A (DENTAURUM J.P. WINKELSTROETER KG; LINDIGKEIT, JUERGEN) 21. Mai 2004 (2004-05-21) * das ganze Dokument * ----- | 1-9 | |
| A,D | DE 41 23 606 A1 (DENTAURUM J. P. WINKELSTROETER KG, 7536 ISPRINGEN, DE) 21. Januar 1993 (1993-01-21) * das ganze Dokument * ----- | 1-9 | |
| A,D | US 2004/109785 A1 (LINDIGKEIT JURGEN) 10. Juni 2004 (2004-06-10) * das ganze Dokument * ----- | 1-9 | |
| A,D | US 4 229 215 A (PROSEN ET AL) 21. Oktober 1980 (1980-10-21) * das ganze Dokument * ----- | 1-9 | |

**RECHERCHIERTE SACHGEBIETE (Int.Cl.7)**

C22C
A61K

Der vorliegende Recherchenbericht wurde für alle Patentansprüche erstellt

| Recherchenort | Abschlußdatum der Recherche | Prüfer |
|---|---|---|
| München | 13. April 2005 | Swiatek, R |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
............................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 05 10 1162

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-04-2005

| Im Recherchenbericht angeführtes Patentdokument | | Datum der Veröffentlichung | Mitglied(er) der Patentfamilie | | Datum der Veröffentlichung |
|---|---|---|---|---|---|
| DE 29909031 | U1 | 05-08-1999 | DE | 19918426 A1 | 26-10-2000 |
| | | | AT | 254440 T | 15-12-2003 |
| | | | AU | 1157000 A | 10-11-2000 |
| | | | DE | 59907822 D1 | 24-12-2003 |
| | | | WO | 0064403 A1 | 02-11-2000 |
| | | | EP | 1173136 A1 | 23-01-2002 |
| WO 2004042098 | A | 21-05-2004 | DE | 10252776 A1 | 22-07-2004 |
| | | | WO | 2004042098 A1 | 21-05-2004 |
| DE 4123606 | A1 | 21-01-1993 | KEINE | | |
| US 2004109785 | A1 | 10-06-2004 | DE | 10226221 C1 | 11-12-2003 |
| US 4229215 | A | 21-10-1980 | DE | 3001126 A1 | 08-01-1981 |
| | | | JP | 56003647 A | 14-01-1981 |

EPO FORM P0461

Für nähere Einzelheiten zu diesem Anhang : siehe Amtsblatt des Europäischen Patentamts, Nr.12/82